# FASCICULE DE BREVET EUROPEEN

(11) **EP 3 328 449 B1**
(45) Date de publication et mention de la délivrance du brevet: **20.11.2019**
(21) Numéro de dépôt: 16760529.4
(22) Date de dépôt: 29.07.2016
(51) Int. Cl.: A61L 9/12, A47G 1/06

(54) **DISPOSITIF DE DIFFUSION**
DIFFUSIONSVORRICHTUNG
DIFFUSION DEVICE

(30) Priorité: 30.07.2015 FR 1557328
(43) Date de publication de la demande: 06.06.2018
(73) Titulaire: Atelier Elio SA, 75011 Paris (FR)
(72) Inventeur: LOVI-VILOREY, Pierre, 75011 Paris (FR); GOURAND, Didier, 94200 Ivry sur Seine (FR); COUTARD, Eve, 75011 Paris (FR); LAMBERCY, Frédéric, 1337 Vallorbe (CH)
(74) Mandataire: Jolly, Christophe
(86) Numéro de dépôt international: PCT/FR2016/051994
(87) Numéro de publication internationale: WO 2017/017394

(56) Documents cités:
- DE-A1-102010 032 122
- FR-A1- 3 020 953
- GB-A- 2 268 798
- US-A1- 2006 285 269
- US-A1- 2011 139 889

## Description

La présente invention se rapporte à un dispositif de diffusion dans l'air d'un composé volatile, de préférence odorant, destiné par exemple à être masqué par un objet décoratif ou un miroir par exemple.

On connaît du brevet FR1401085 de la demanderesse un dispositif de diffusion comprenant un boîtier logeant un circuit de circulation d'air dans lequel est agencé un moyen de ventilation et des flacons de produits bactérien et diffuseur d'odeurs. Le boîtier comprend des parois latérales pourvues d'orifices de passage d'air. En fonctionnement, le moyen de ventilation permet une circulation d'air chargée en particules provenant des flacons de produits bactériens et diffuseurs d'odeurs au travers des orifices de sortie des parois latérales, ce qui permet de répandre lesdits produits dans l'atmosphère environnante dans laquelle le dispositif de diffusion est installé.

Si ce type de système est simple à mettre en œuvre, il ressort toutefois que la diffusion de l'air ne s'effectue pas de manière uniforme au travers des différents orifices de sortie d'air, ce qui ne permet pas d'avoir une diffusion uniforme de l'air sur la surface de diffusion souhaitée, la pression en air diminuant au fur et à mesure que les orifices sont éloignés du moyen de ventilation.

De plus, il s'avère également que la diffusion du composé volatile odorant ne s'effectue pas sur une large zone d'espace, ne permettant ainsi pas une bonne propagation du composé volatile odorant de manière adéquate.

L'invention a notamment pour but d'apporter une solution simple, efficace et économique aux problèmes de l'art antérieur décrit précédemment.

A cet effet, elle propose un premier dispositif de diffusion d'une substance volatile comprenant :
- un boîtier définissant intérieurement un circuit de circulation d'air entre au moins une entrée d'air et au moins une sortie d'air,
- un moyen de ventilation agencé dans le circuit d'air du boîtier et apte à faire circuler un flux d'air depuis l'entrée jusqu'à la sortie d'air, et
- une zone de réception agencée dans le circuit d'air en aval du moyen de ventilation et destinée à recevoir un substrat retenant une substance volatile, de préférence odorante,
caractérisé en ce que le circuit d'air comprend au moins une chambre agencée en aval de ladite zone de réception et en amont d'une sortie d'air du boîtier.

De préférence, le dispositif comprend trois chambres qui sont indépendantes fluidiquement les unes des autres, chacune des chambres étant agencée en aval de ladite zone de réception et communiquant en aval avec sa propre sortie d'air différente des autres sorties d'air, une première et une deuxième chambres communiquant avec des sorties d'air projetant de l'air dans des sens opposés, et une troisième chambre communiquant avec une sortie d'air qui projette de l'air dans une direction perpendiculaire à la direction de projection d'air de sortie des première et deuxième chambres.

L'intégration de trois chambres de diffusion chacune reliée à sa propre sortie d'air permet de mieux diffuser la substance volatile dans une large zone d'espace à l'extérieur du boîtier. La diffusion d'air peut ainsi se faire de manière uniforme dans l'air extérieur évitant une impression désagréable de trop forte concentration à certains endroits d'une pièce comme cela est le cas avec les appareils de la technique antérieure.

Selon une autre caractéristique, la section de la ou des chambres augmente de l'amont vers l'aval.

Les sections des chambres du circuit d'air peuvent avoir une section qui augmente en allant vers l'aval ce qui permet de réaliser, en fonctionnement, une accumulation de la substance volatile dans la chambre. De plus, le fait que la chambre débouche directement dans une sortie d'air du boîtier permet de réaliser une diffusion directe de l'air chargé de produit volatile à l'extérieur du boîtier et sur une grande surface de diffusion du fait de l'augmentation de la section de la chambre en allant vers l'aval.

Il peut être souhaitable que la section d'au moins une ou de chaque sortie d'air soit inférieure à la section aval de la chambre associée à la sortie d'air. De cette manière, on peut réaliser une accumulation de la substance volatile dans la chambre pour un débit du moyen de ventilation au moins égal à une valeur seuil, ce qui permet d'assurer une bonne diffusion du parfum sur toute la sortie d'air.

Selon encore une autre caractéristique, le boîtier peut comprendre une première paroi de fond et une seconde paroi de fond, qui peuvent être sensiblement parallèles l'une à l'autre et entre lesquelles s'étendent au moins une première, une deuxième et une troisième parois latérales, un bord de chacune des première, deuxième et troisième parois latérales définissant avec un bord de l'une des première et seconde parois de fond, respectivement une fente de sortie d'air des première, deuxième et troisième chambres. La première paroi latérale et la deuxième paroi latérale peuvent être sensiblement parallèles l'une à l'autre et être agencées en vis-à-vis. La troisième paroi latérale peut être sensiblement perpendiculaire aux première et deuxième parois latérales. Les première et seconde parois de fond peuvent être perpendiculaires aux première, deuxième et troisième parois latérales.

Les sorties d'air sont ainsi formées par des fentes entre les première, deuxième et troisième parois latérales et l'une des première et seconde parois de fond du boîtier, ce qui permet de diffuser l'air chargé en particules volatiles provenant du substrat sur toute l'étendue des fentes de sortie d'air.

Préférentiellement, chaque sortie d'air s'étend tout le long d'un côté du boîtier de manière à ce que l'air puisse se diffuser sur tout un côté du boîtier et encore améliorer la diffusion, en fonctionnement, de la substance volatile.

La présente description concerne également un second dispositif comprenant :
- un boîtier comprenant intérieurement un circuit de circulation d'air entre au moins une entrée d'air et au moins une sortie d'air,
- un moyen de ventilation agencé dans le circuit d'air et apte à faire circuler un flux d'air depuis l'entrée jusqu'à la sortie d'air, et
- une zone de réception agencée dans le circuit d'air en aval du moyen de ventilation et destinée à recevoir un substrat retenant une substance volatile, de préférence odorante,
ce dispositif comprenant en outre :
- un chemin de déplacement du substrat formé à l'intérieur du boîtier et s'étendant entre une première ouverture et une seconde ouverture formées dans des parois du boîtier et accessibles depuis l'extérieur du boîtier, et
- des moyens de guidage à déplacement du substrat le long du chemin de déplacement lequel intercepte le circuit d'air au niveau de la zone de réception.

Ainsi, le boîtier comprend un chemin de déplacement du substrat à l'intérieur du boîtier entre une première et une seconde ouverture de manière à ce qu'un opérateur puisse insérer manuellement un substrat, depuis l'extérieur du boîtier, par la première ouverture par exemple et le récupérer après utilisation (plusieurs mois par exemple) par la seconde ouverture, par exemple.

On comprend que les caractéristiques du second dispositif de diffusion peuvent être intégrées au premier dispositif de diffusion de manière à former un troisième dispositif de diffusion comme cela apparaît à titre d'exemple dans la description détaillée.

Les caractéristiques indiquées ci-après peuvent être intégrées à au moins l'un du premier dispositif, du second dispositif et du troisième dispositif.

Selon une autre caractéristique, le chemin de déplacement peut être dimensionné de manière à loger au moins trois substrats agencés bout à bout dans une première position de stockage, une position de service dans laquelle le substrat est dans la zone de réception, et une deuxième position de stockage.

Dans cette configuration, lorsque le chemin de déplacement est dimensionné de manière à avoir une longueur égale ou voisine de celle de trois substrats et loge trois substrats, l'insertion d'un substrat dans la première ouverture induit les déplacements suivants :
- le substrat inséré par la première ouverture est positionné dans la première position de stockage ;
- le substrat qui est dans la première position de stockage est déplacé dans la position de service ;
- le substrat qui est dans la position de service est déplacé dans la deuxième position ; et
- le substrat qui est dans la deuxième position de stockage est éjecté du boîtier.

Préférentiellement, la première ouverture est formée dans la première paroi latérale et la seconde ouverture est formée dans la deuxième paroi latérale.

Dans une configuration particulière de l'invention, les moyens de guidage comprennent un rail rectiligne en U s'étendant entre la première ouverture et la seconde ouverture et délimitant intérieurement le chemin de déplacement d'un substrat.

Le rail de guidage peut comprendre un organe de guidage rectiligne s'étendant entre la première ouverture et la seconde ouverture, cet organe étant apte à coopérer avec un organe de guidage complémentaire formé sur chacun des substrats.

Plus spécifiquement, l'organe de guidage peut comprendre une nervure rectiligne formée dans une branche du rail en U.

Le chemin de déplacement peut comprendre au niveau de la première ouverture un organe élastique anti-retour autorisant l'introduction d'un substrat dans la première ouverture et empêchant son retrait du chemin de déplacement au travers de la première ouverture.

Le chemin de déplacement peut comprendre au niveau de la seconde ouverture un organe élastique de freinage configuré pour freiner la sortie d'un substrat par la seconde ouverture.

De cette manière, l'introduction d'un substrat dans le chemin de déplacement n'est possible que dans la première ouverture et la sortie d'un substrat n'est possible que dans la seconde ouverture.

Le rail peut être coiffé par un couvercle rectiligne, de préférence en U, portant à une extrémité l'organe élastique anti-retour et à une autre extrémité l'organe élastique de freinage.

Egalement, l'organe élastique anti-retour et l'organe élastique de freinage peuvent comprendre une lame élastique, qui peut être de même forme.

L'une des première et seconde parois de fond peut comprendre une ouverture débouchant dans la zone de réception, un porte-substrat pouvant être conformé de manière à pouvoir être inséré à force dans l'ouverture pour la mise en position du substrat dans la zone de réception.

La zone de réception prévue pour loger le porte-substrat portant lui-même le substrat intégrant le composé volatile odorant, est ainsi accessible depuis l'extérieur, ce qui permet de réaliser de manière simple et rapide une opération de maintenance sur le dispositif de diffusion lorsque le substrat doit être remplacé.

Le boîtier peut également comprendre une enceinte interne isolée fluidiquement du circuit d'air et à l'intérieur de laquelle sont logés des moyens de commande du ventilateur et des moyens d'alimentation électrique, ces moyens étant ainsi isolés du circuit d'air.

Selon une autre caractéristique, au moins un cadre support peut être fixé sur la première paroi et comporter des moyens de support formés en saillie sur au moins une partie du contour du boîtier et aptes à coopérer avec des moyens d'accrochage d'un objet.

L'invention concerne également un ensemble comprenant un dispositif tel que décrit ci-dessus et un substrat conformé de manière à pouvoir être agencé dans ladite zone de réception.

Avantageusement, le substrat a une forme sensiblement parallélépipédique, par exemple rectangle, et comprend une pluralité de orifices débouchant sur deux faces opposées du substrat.

Au moins certains des orifices peuvent avoir une section se réduisant depuis une extrémité vers l'autre extrémité.

Avantageusement le substrat est agencé dans le logement de réception de sorte que la section se réduit de l'amont vers l'aval.

Selon une autre caractéristique, le substrat peut présenter une section de forme générale rectangulaire comportant à un premier sommet une rampe inclinée en oblique et à un second sommet un renfoncement.

L'invention concerne encore un substrat, tel qu'un substrat pour dispositif de diffusion d'une substance volatile odorante, dans lequel le substrat est solide, intègre une substance volatile et comprend une face amont et une face aval en vis-à-vis entre lesquelles s'étendent des orifices pour la circulation d'air au travers du substrat, le substrat comportant une première extrémité d'insertion dans un dispositif de diffusion et une seconde extrémité opposée, la première extrémité comprenant une rampe destinée à coopérer avec un organe élastique anti-retour et la seconde extrémité comprenant un renfoncement dont une surface est destinée à former une surface de butée de l'organe anti-retour.

Selon une autre caractéristique de l'invention, le substrat a une forme sensiblement parallélépipédique. Selon un plan de coupe intercalé entre les faces amont et aval et passant par les première et seconde extrémités, le substrat a une forme de parallélogramme, la rampe étant située au niveau d'un premier sommet agencé du côté de la première extrémité du substrat et le renfoncement étant situé au niveau d'un second sommet agencé du côté de la seconde extrémité du substrat.

La forme du substrat peut être sensiblement rectangulaire et/ou le premier sommet et le second sommet peuvent être reliés par une même arête s'étendant entre les première et seconde extrémités du substrat.

La rampe peut être sensiblement plane et inclinée en oblique.

L'invention sera mieux comprise et d'autres détails, avantages et caractéristiques de l'invention apparaîtront à la lecture de la description suivante faite à titre d'exemple non limitatif, en référence aux dessins annexés dans lesquels :
- la figure 1 est une vue schématique en perspective depuis l'extérieur dispositif de diffusion selon l'invention ;
- la figure 2 est une vue schématique en perspective du dispositif de la figure 1 avec éclatement des organes destinés à supporter un élément décoratif par exemple ;
- la figure 3 est une vue schématique en perspective isolé d'organe de support de la figure 2 ;
- les figures 4 à 6 sont des vues schématiques en perspective selon des plans de coupes différents du dispositif de diffusion de la figure 1 ;
- la figure 7 est une vue schématique en perspective et à plus grande échelle de la zone délimitée en pointillée sur la figure 6 ;
- la figure 8 est une vue schématique en coupe d'une sortie d'air du dispositif de diffusion selon l'invention ;
- la figure 9 est une vue schématique du dispositif de diffusion selon l'invention, le substrat retenant une substance volatile étant en position partiellement sortie ;
- les figures 10 et 11 sont des vues schématiques d'une réalisation pratique possible de l'invention ;
- les figures 12 et 13 sont des vues schématiques des orifices dans un substrat retenant une substance volatile ;
- la figure 14 est une vue schématique en perspective d'une seconde réalisation du dispositif selon l'invention ;
- les figures 15 à 17 sont des vues schématiques en perspective de la seconde réalisation du dispositif ;
- les figures 18 et 19 sont des vues schématiques du rail de guidage des substrats selon la seconde réalisation ;
- la figure 20 est une vue schématique en perspective d'un organe élastique utilisé pour l'insertion des substrats ;
- les figures 21 et 22 sont des vues schématiques de côté d'extrémités opposées du rail de guidage de la figure 19 ;
- la figure 23 est une vue schématique en perspective de l'extrémité du rail représentée en figure 21 ;
- la figure 24 est une vue schématique en perspective de l'extrémité du rail représentée en figure 22 ;
- les figures 25 et 26 sont des vues schématiques d'un substrat selon une variante de réalisation.

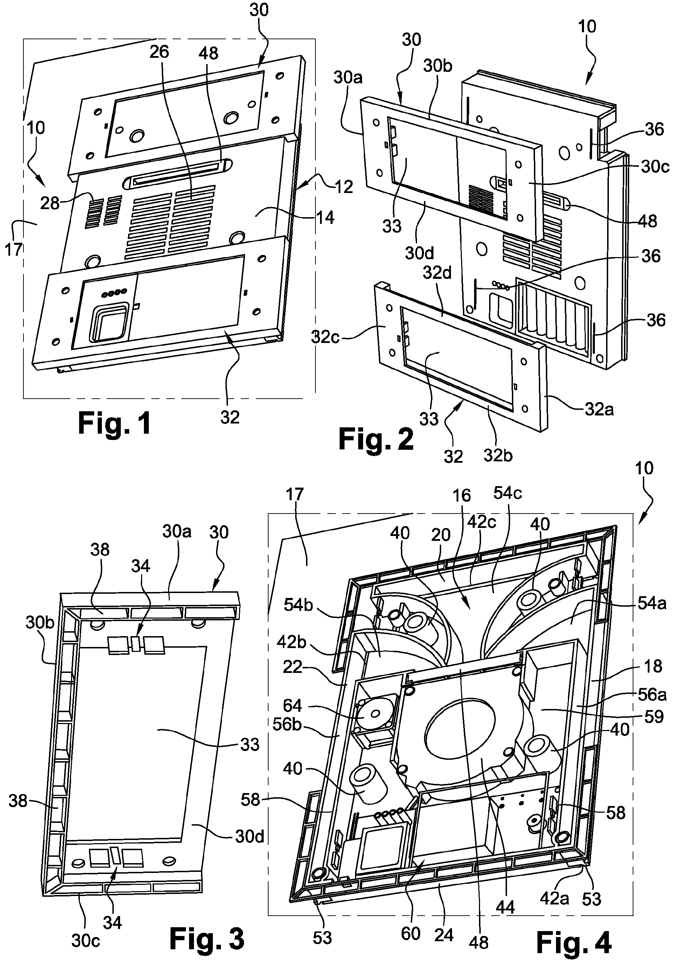

On se réfère simultanément aux figures 1, 2, 4, 5, 6 et 10 qui représentent un dispositif de diffusion 10 d'une substance volatile selon l'invention comprenant un boîtier 12, de forme générale correspondant à un parallélépipède rectangle, qui comporte une première paroi 14 de fond et une seconde paroi 16 de fond. Ces première 14 et seconde 16 parois de fond sont planes, agencées à distance l'une de l'autre et sensiblement parallèlement l'une à l'autre. La seconde paroi 16 est une paroi destinée à venir en contact avec un support vertical 17 tel qu'un mur intérieur d'une habitation tandis que la première paroi 14 est destinée à venir en vis-à-vis d'un objet décoratif ou un miroir par exemple. Des parois latérales 18, 20, 22, 24 s'étendent entre la première paroi 14 de fond et la seconde paroi 16 de fond et définissent ensemble le pourtour extérieur du boîtier 12. Les parois latérales 18, 20, 22, 24 sont planes et sensiblement perpendiculaires aux première 14 et seconde 16 parois de fond. On distingue ainsi la présence d'une première paroi latérale 18 et d'une deuxième paroi latérale 22 parallèles l'une à l'autre et en vis-à-vis l'une de l'autre ainsi que d'une troisième paroi latérale 20 et d'une quatrième paroi latérale 24 parallèles l'une à l'autre et en vis-à-vis l'une de l'autre. Les première 18 et deuxième 24 parois latérales sont perpendiculaires aux troisième 20 et quatrième 24 parois latérales.

La première paroi de fond 14 comprend deux grilles 26, 28 d'ouvertures de forme rectangulaire formant pour l'une une grille d'entrée d'air 26 d'un circuit d'air et pour l'autre une grille 28 de passage de sons audibles comme cela apparaîtra plus clairement dans la suite de la description. La première grille 26 est agencée de manière sensiblement centrale sur la première paroi 14.

Comme représenté aux figures 1, 2 et 3, le dispositif de diffusion 10 comprend des moyens de support destinés à supporter un objet décoratif ou un miroir. A cette fin, les moyens de support comprennent un premier cadre support 30 monté à une première extrémité du boîtier 12 et appliqué sur la première paroi 14 et un second cadre support 32 monté à une seconde extrémité du boîtier 12 opposée à la première extrémité et appliqué sur la première paroi 14. Chaque premier cadre 30 et second cadre 32 est formé d'une seule pièce et comprend un premier 30a, 32a, un second 30b, 32b, un troisième 30c, 32c et un quatrième 30d, 32d montants successifs définissant ensemble un rectangle délimitant intérieurement une ouverture centrale 33. Le premier montant 30a, 32a et le troisième montant 30c, 32c de chaque cadre 30, 32 portent des organes d'encliquetage élastique 34 dans des fentes 36 de la première paroi 14. Les premiers 30a, 32a, seconds 30b, 32b et troisièmes 30c, 32c montants comprennent une succession d'évidements rectangulaires 38 destinés à recevoir des moyens d'accrochage d'un objet décoratif ou d'un miroir. On remarque que chaque cadre 30, 32 est dimensionné de manière à ce que les évidements 38 soient agencés en saillie sur le pourtour du boîtier 12 afin de permettre un engagement des moyens d'accrochage d'un objet décoratif dans les évidements.

Les première 14 et seconde 16 parois de fond sont reliées par des passages tubulaires 40 destinés à recevoir des vis de fixation du dispositif de diffusion sur un support d'une habitation tel qu'un mur par exemple. Comme cela est visible sur la figure 1, deux passages tubulaires 40 traversent l'ouverture 33 du premier cadre 30 tandis que les deux autres passages tubulaires 40 traversent la première paroi de fond 14 et la seconde paroi de fond 16 de manière à déboucher sur la première paroi de fond 14 entre les premier 30 et second 32 cadres support. L'agencement des passages tubulaires 40 est déterminé en fonction de la place disponible dans le boîtier 12 pour le montage des éléments nécessaires au fonctionnement du dispositif de diffusion 10 comme cela apparaîtra plus clairement dans la suite de la description.

Le boîtier 12 comprend un circuit d'air dont l'entrée d'air est formée par la grille 26 de la première paroi de fond 14 du boîtier 12 comme indiqué précédemment et une pluralité de sorties 42a, 42b, 42c d'air, une première sortie d'air 42a, une deuxième sortie d'air 42b et une troisième sortie d'air 42c. Le circuit d'air comprend un moyen de ventilation 44 aspirant de l'air depuis l'extérieur par la grille 26 et rejetant l'air au travers d'un substrat 46 ou cartouche agencé en aval du moyen de ventilation 44. Le substrat 46 est porté par un porte-substrat 48 qui est inséré dans une zone de réception 50 agencée immédiatement en aval du moyen de ventilation. Cette zone de réception 50 est accessible depuis l'extérieur du boîtier 12, par l'intermédiaire d'une ouverture 52 de la première paroi de fond 14 de forme complémentaire de celle du porte-substrat 48 et débouchant dans la zone de réception 50. L'ouverture 52 de la première paroi de fond 14 et le porte-substrat 48 sont dimensionnés l'un par rapport à l'autre de manière à ce que le porte-substrat 48 soit inséré à force dans l'ouverture 52 de la première paroi de fond 14 pour venir se loger dans la zone de réception 50 du circuit d'air et puisse y être maintenu. Le porte-substrat 48 est formé par un cadre rectangulaire en matière rigide et dans lequel le substrat 46 peut être introduit avant que l'ensemble porte-substrat 48 et substrat 46 soit mis en position dans le circuit d'air (figures 1, 6, 7 et 9). Lorsque le boîtier 12 est accroché sur un mur, par exemple, la seconde paroi de fond 16 est agencée en regard du mur et la première paroi de fond 14 face est agencée à l'opposée, ce qui rend permet un accès à l'ouverture 52 pour pouvoir réaliser une maintenance sur le dispositif, permettant ainsi d'enlever le porte-substrat 48 pour retirer le substrat usagé et y insérer un nouveau substrat 46.

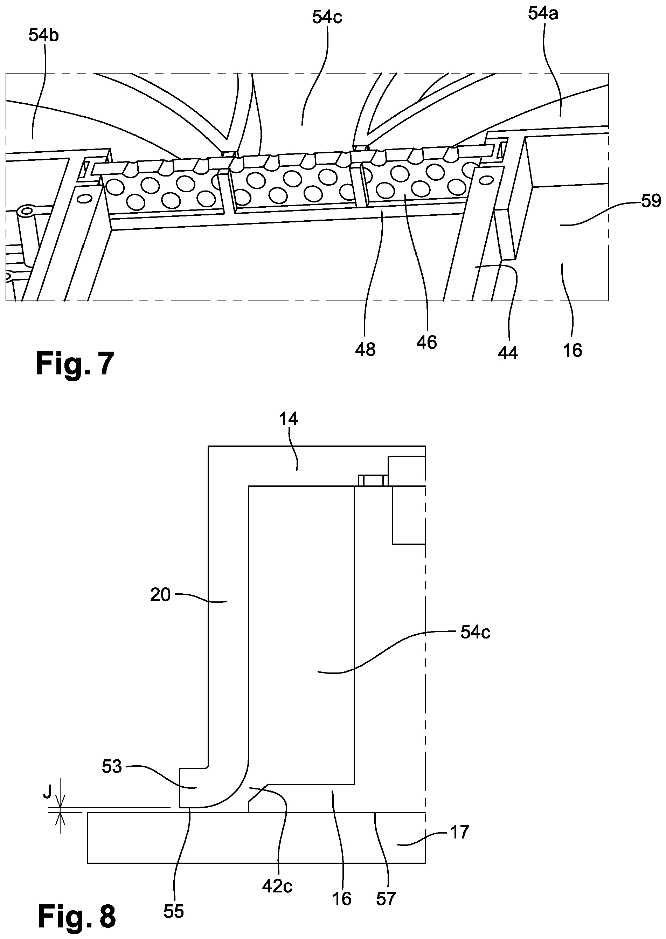

Le circuit d'air comprend trois chambres, une première chambre 54a, une deuxième chambre 54b et une troisième chambre 54c de diffusion agencées en aval de la zone de réception 50 du substrat 46, la première chambre 54a communiquant avec la première sortie d'air 42a, la deuxième chambre 54b communiquant avec la deuxième sortie d'air 42b et la troisième chambre 54c communiquant avec la troisième sortie d'air 42c du boîtier 12. La première sortie d'air 42a est formée par une fente rectiligne délimitée entre un bord de la première paroi latérale 18 du boîtier 12 et un bord périphérique de la seconde paroi de fond 16. La deuxième sortie d'air 42b est formée par une fente rectiligne délimitée entre un bord de la deuxième paroi latérale 22 du boîtier 12 et un bord périphérique de la seconde paroi de fond 16. La troisième sortie d'air 42c est formée par une fente rectiligne délimitée entre un bord de la troisième paroi latérale 20 du boîtier 12 et un bord périphérique de la seconde paroi de fond 16. Chaque fente 42a, 42b, 42c s'étend ainsi tout le long du bord d'une paroi latérale 18, 20, 22 et du bord périphérique de la seconde paroi 16 (figure 8).

Comme cela est plus particulièrement visible sur la figure 8, la troisième paroi latérale 20 porte un rebord 53 s'étendant vers l'extérieur du boîtier 12 dans une direction perpendiculaire à la troisième paroi latérale 20. Ce rebord 53 est conformé de manière à ce que sa face 55 orientée en vis-à-vis du mur 17 soit agencée à distance de celui-ci. Plus particulièrement, dans une direction perpendiculaire à la seconde paroi de fond 16, la face 55 est espacée d'un jeu J de la face externe 57 de la seconde paroi de fond 16, ce qui permet l'écoulement d'air vers l'extérieur sur la périphérie du boîtier 12. Ce jeu J est ici inférieur à la largeur de la deuxième fente 42b. Les première 18 et deuxième 22 parois latérales peuvent également comprendre un rebord identique au rebord 53 de la paroi latérale et définissant un jeu J avec la face externe 57 de la seconde paroi de fond 16, comme décrit ci-dessus.

Comme représenté chacune des première 54a, deuxième 54b et troisième 54c chambres présente une forme qui s'évase depuis son extrémité amont jusqu'à son extrémité aval. Ainsi, la section de chaque chambre 54a, 54b, 54c augmente depuis son extrémité amont jusqu'à son extrémité aval qui communique avec une sortie d'air 42a, 42b, 42c.

Plus particulièrement, la première 54a et la deuxième 54b chambres communiquent avec des fentes d'air 42a, 42c projetant de l'air dans des sens opposées selon une première direction (flèches A). La troisième chambre 54c communique avec la fente 42c qui projette de l'air dans une seconde direction (flèche B) sensiblement perpendiculaire à la direction de projection des fentes 42a, 42b par exemple dans un sens orienté vers le haut lorsque le boîtier est positionné de manière adéquate. Selon l'invention, chaque chambre 54a, 54b, 54c est associée à une fente 42a, 42b, 42c de sortie d'air indépendante des autres chambres 54a, 54b, 54c et fentes 42a, 42b, 42c de sorties d'air. On remarque que la troisième chambre 54c est séparée de la première chambre 54a et de la deuxième chambre 54c par deux passages tubulaires 40, 40.

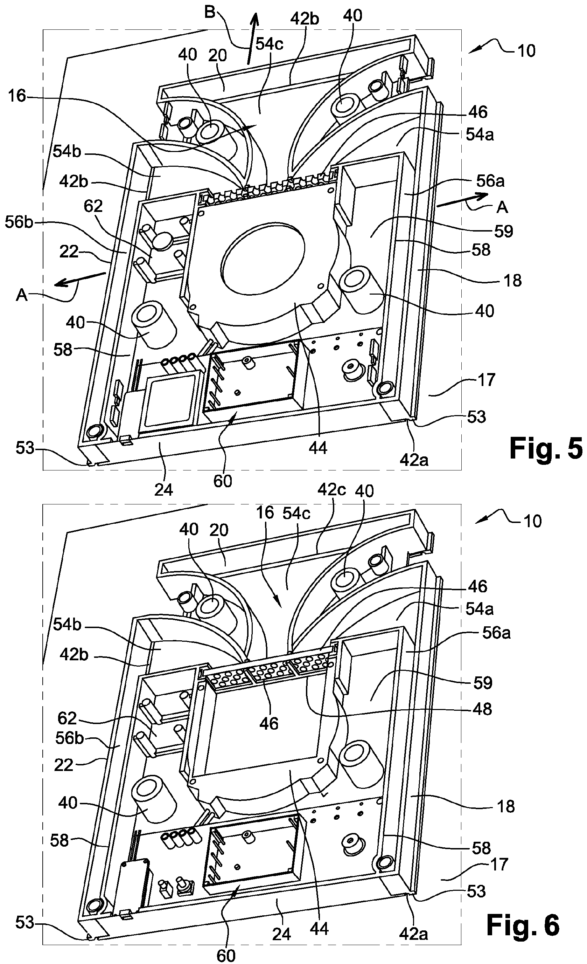

Comme cela est visible sur les figures 5 et 6 plus particulièrement, les portions aval des première 54a et deuxième 54b chambres comprennent un canal aval 56a, 56b destiné à réaliser une répartition du flux tout le long de la fente 42a, 42b de sortie d'air. Le canal 56a est délimité par la première paroi latérale 18 et une cloison 58 en vis-à-vis délimitant une partie d'une enceinte interne 59. Le canal 56b est délimité par la deuxième paroi latérale 22 et une cloison 58 en vis-à-vis délimitant une partie de l'enceinte interne 59. On remarque qu'ainsi configuré, la première chambre 54a et son canal 56a sont symétriques de la deuxième chambre 54b et de son canal 56b par rapport à un plan médian de la troisième chambre 54c et perpendiculaire à la seconde paroi de fond 16. Plus généralement, le circuit d'air est symétrique par rapport au plan précité.

L'enceinte interne 59 est délimitée par les cloisons 58 en vis-à-vis, la quatrième paroi latérale 24 et l'enveloppe extérieur ou carter du moyen de ventilation 44. Cette enceinte est isolée fluidiquement du circuit d'air et loge des moyens de commande du débit du ventilateur et des moyens d'alimentation électrique 60. L'enceinte interne 59 peut également comprendre un logement 62 d'un haut-parleur 64 relié à des moyens de traitement d'un signal numérique issu d'un fichier audio provenant d'une clef USB, par exemple, qui peut être branchée sur un connecteur accessible depuis l'extérieur du boîtier (non représenté).

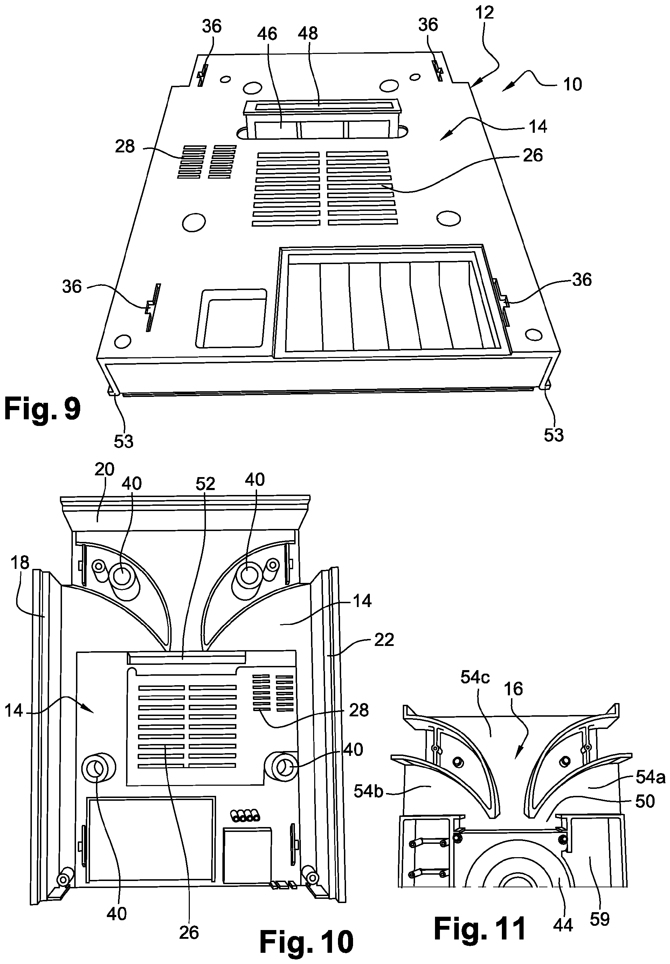

Dans la configuration représentée aux figures 10 et 11, les première, 18, deuxième 22 et troisième 20 parois latérales définissant avec la seconde paroi de fond 16 lesdites première, 42a, deuxième 42b et troisième 42c fentes de sortie d'air, sont solidaires de la première paroi de fond 14, tandis que la quatrième paroi latérale 24 est solidaire de la seconde paroi de fond 16 (figures 10 et 11). Les passages tubulaires 40 sont également solidaires de la première paroi de fond 14.

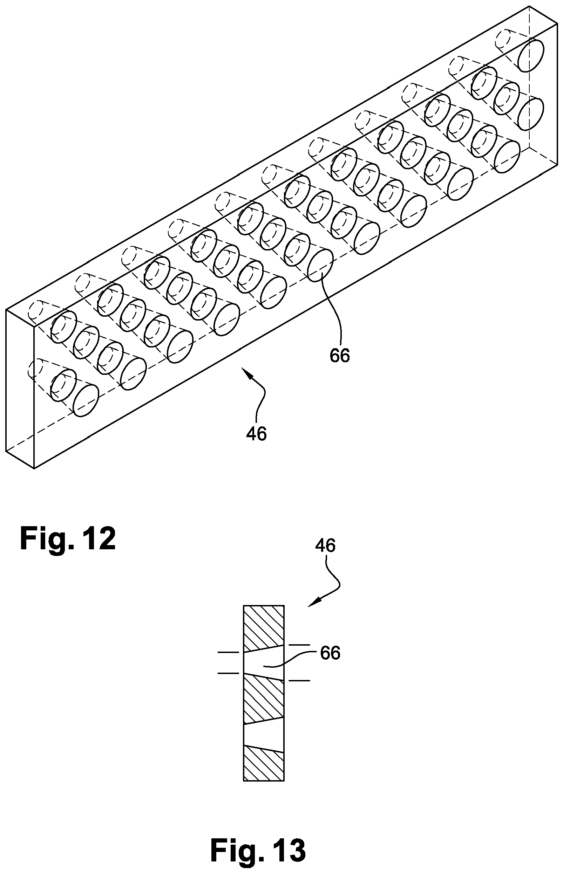

Comme cela est plus particulièrement visible sur les figures 7, 12 et 13, le substrat 46, incorporant une substance volatile, a l'aspect d'une plaquette présentant une forme parallélépipédique rectangle ou pavé droit et est traversé de part en part en entre ses deux plus grandes faces par des orifices 66 ayant une forme tronconique. Lorsque le substrat 46 est monté dans le porte-substrat 48 et que ce dernier est agencé dans la zone de réception 50 du circuit d'air, les orifices 66 sont orientés de manière à ce que la section de chacun de ceux-ci se réduise dans le sens amont/aval.

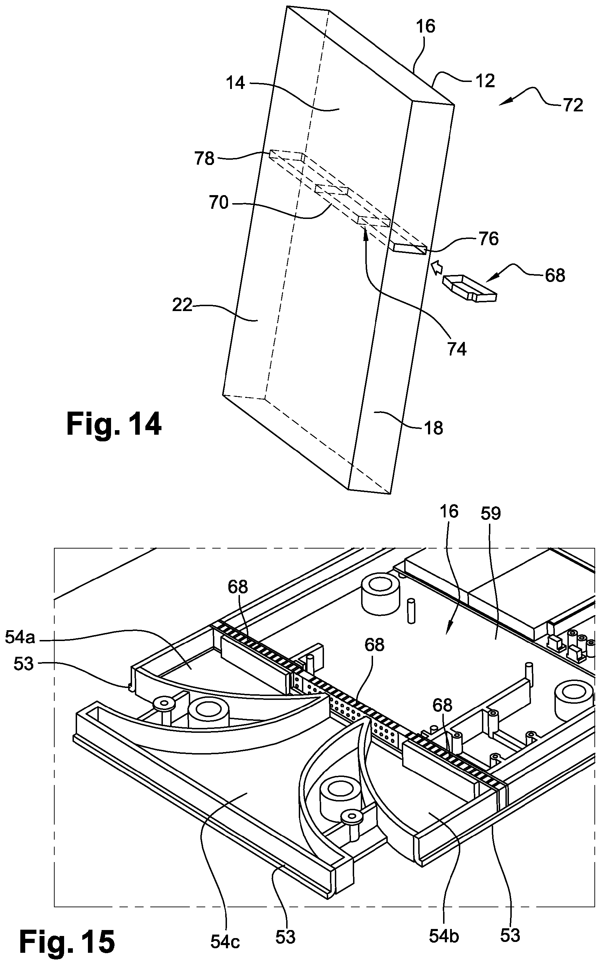

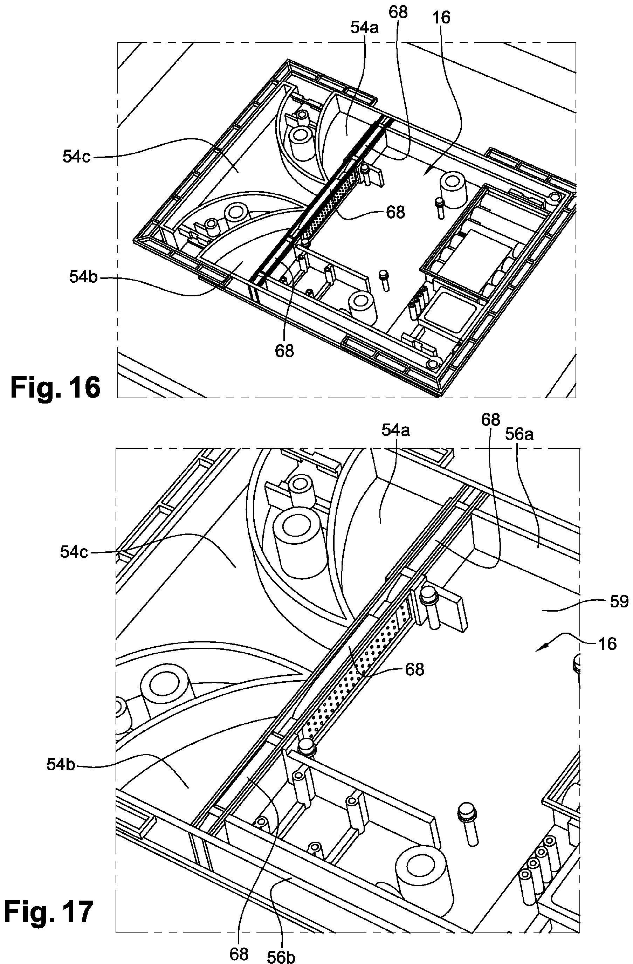

Les figures 14 et suivantes représentent une variante d'intégration du substrat 68 ou cartouche dans la zone de réception 70 du dispositif 72, précédemment. Ainsi, dans cette variante, la première paroi de fond 14 ne comprend pas d'ouverture débouchant dans la zone de réception 70 mais comprend un chemin de déplacement 74 intégrant la zone de réception 70 et qui est formé à l'intérieur du boîtier 12 et qui s'étend entre une première ouverture 76 formée sur la première paroi latérale 18 et une seconde ouverture 78 formée dans la deuxième paroi latérale 22 (figure 14). Ce chemin de déplacement 74 est sensiblement rectiligne et est perpendiculaire aux première 18 et deuxième 22 parois latérales. Comme cela est représenté sur les figures 15 à 17, le chemin de déplacement 74 intercepte le circuit d'air au niveau de la zone de réception 70 de manière à ce qu'un substrat 68 introduit dans la première ouverture 76, amené jusque dans la zone de réception 70 puisse être traversé par l'air du le circuit d'air.

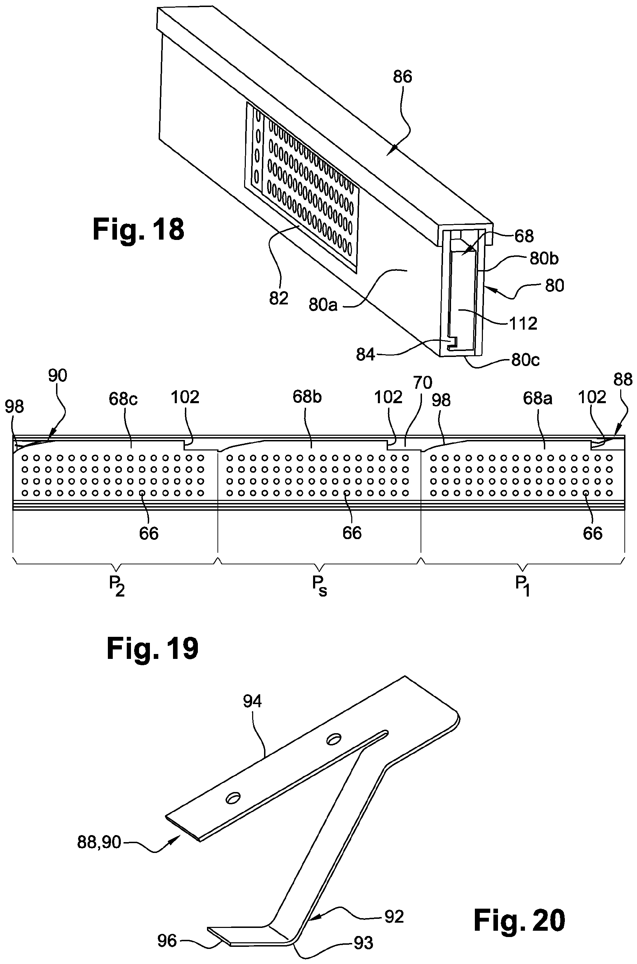

Le chemin de déplacement 74 est dimensionné de manière à pouvoir loger une pluralité de substrats 68, dans l'exemple représenté trois substrats 68, agencés bout à bout. Dans ce cas, un premier substrat 68a est agencé dans une première position de stockage P₁ dans laquelle le premier substrat 68a n'est pas dans le circuit d'air, un deuxième substrat 68b est agencé dans une position de service P_{S} dans laquelle le substrat est dans la zone de réception 70, et un troisième substrat 68c est agencé dans une deuxième position de stockage P₂ dans laquelle le substrat 68c n'est pas dans le circuit d'air. Ainsi, le chemin de stockage comprend trois positions successives une première position de stockage P₁, une position de service P_{S}, et une deuxième position de stockage P₂ (figure 19). On comprend aisément qu'un substrat 68 inséré dans la première ouverture 76 du chemin de déplacement 74, celui-ci comprenant déjà trois substrat 68a, 68b, 68c induit les déplacements suivants :
- le substrat 68 inséré par la première ouverture 76 est positionné dans la première position de stockage P₁ ;
- le substrat 68a qui est dans la première position de stockage P₁ est déplacé dans la position de service P_{S} ;
- le substrat 68b qui est dans la position de service P_{S} est déplacé dans la deuxième position P₂ ; et
- le substrat 68c qui est dans la deuxième position de stockage P₂ est éjecté du boîtier 12.

Comme cela apparaîtra ultérieurement, la première position de stockage P₁ correspond à une position de stockage d'un substrat 68a ou cartouche à l'état neuf et la seconde position de stockage P₂ correspond à une position de stockage d'un substrat 68c à l'état usagé, c'est-à-dire qui est passé par la position de service P_{S}.

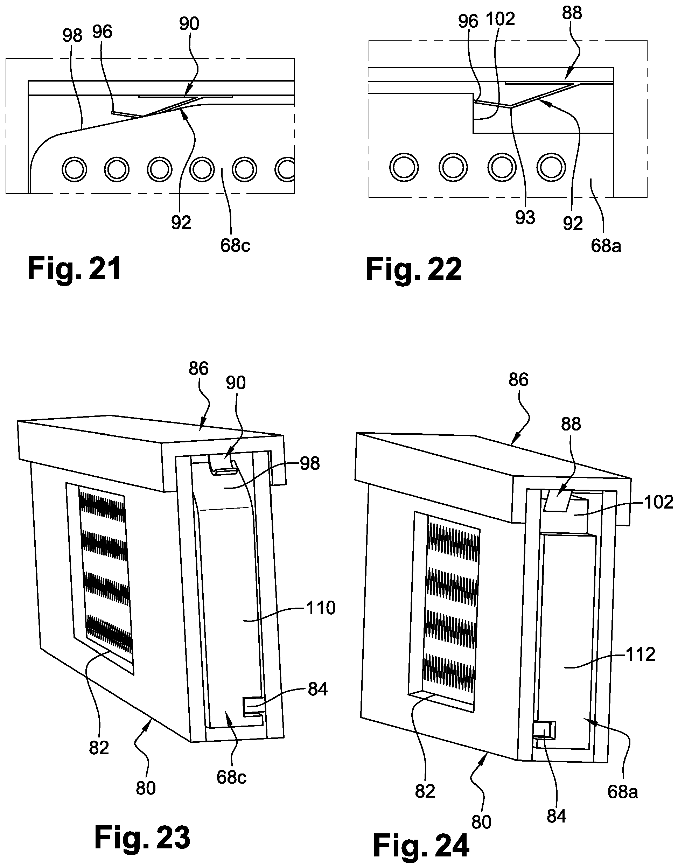

Le dispositif comprend des moyens de guidage à déplacement des substrats 68 le long du chemin de déplacement 74 (figures 18, 23 et 24). Ces moyens de guidage comprennent un rail 80 rectiligne s'étendant entre la première ouverture 76 et la seconde ouverture 78 et est logé à l'intérieur du boîtier 12 entre la première paroi de fond 14 et la seconde paroi de fond 16. Ce rail 80 délimite intérieurement le chemin de déplacement 74 des substrats 68. Il est ici dimensionné de manière pouvoir loger intégralement les substrats 68. Le rail 80 a une forme en U comportant une première branche 80a et une seconde branche 80b sensiblement parallèles l'une à l'autre et reliées entre elles par une paroi de jonction 80c. La première branche 80a comprend une lumière 82 agencée en vis-à-vis d'une lumière 82 de la seconde branche 80b, les lumières 82 étant positionnées sur le rail 80 de manière à ce que le circuit d'air circule au travers de ces lumières 82. La position de ces lumières 82 correspond ainsi à la position de service P_{S} d'un substrat 68.

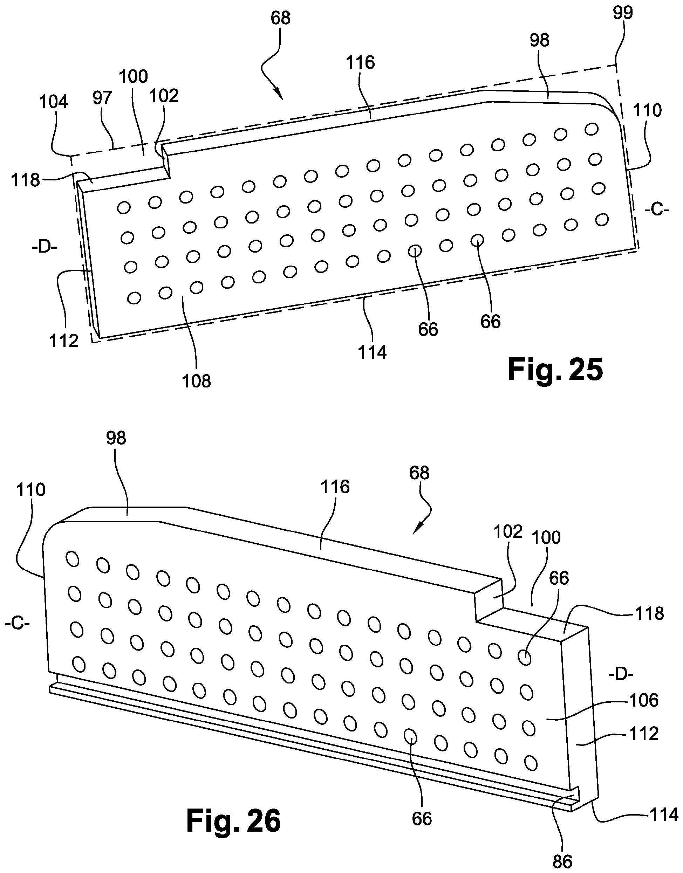

La première branche 80a comprend un organe de guidage 84 formé par une nervure rectiligne s'étendant parallèlement à la paroi de jonction 80c. Cette nervure 84 coopère avec une rainure 86 formée sur la face amont de chacun des substrats 68 (figures 25 et 26). La rainure pourrait être formée sur la face aval du substrat 68 pour coopérer, dans ce cas, avec une nervure de la seconde branche 80b du rail 80.

Le rail 80 est coiffé par un couvercle 86 rectiligne ayant la forme d'un profilé en U. Ce couvercle 86 limite l'écartement des branches 80a, 80b du rail 80 en U. Il porte deux organes élastiques, l'un 88 agencé dans le chemin de déplacement 74 au niveau de la première ouverture 76 et assurant une fonction anti-retour et un autre 90 agencé dans le chemin de déplacement 74 au niveau de la seconde ouverture 78 et assurant une fonction de freinage (figures 19 et 21 à 24).

L'organe élastique anti-retour 88 et l'organe élastique de freinage 90 comprennent chacun une lame à ressort 92 ou lame élastique (figure 20) reliée à une embase 94 fixée par boulonnage sur le couvercle 86. La lame élastique 92 présente une forme en V comportant un sommet ou coude 93. Comme cela est représenté sur la figure 19, la lame élastique anti-retour est agencée de manière à ce que son extrémité libre 96 soit dirigée vers la seconde ouverture 78 et la lame élastique de freinage 92 est également agencée de manière à ce que son extrémité libre 96 soit orientée vers la seconde ouverture 78.

Chaque substrat 68 à une forme parallélépipédique rectangle et présente une section de forme générale rectangulaire représenté en contours pointillés 97 (figure 25) selon un plan de coupe intercalé entre une face amont 106 et une face aval 108 et passant par une première extrémité C d'insertion du substrat dans le dispositif et une seconde extrémité D opposée (figures 25 et 26). Il comprend une rampe 98 plane inclinée en oblique située au niveau d'un premier sommet 99 du contour rectangulaire 97 agencé du côté de la première extrémité C du substrat 68, et un renfoncement 100 comportant un épaulement 102 formé au niveau d'un second sommet 104 adjacent au premier sommet et situé du côté de la seconde extrémité D du substrat 68. Comme représenté, le premier sommet 99 et le second sommet 104 sont reliés par une même arête s'étendant entre les première C et seconde D extrémités du substrat 68.

Les faces amont 106 et aval 108 du substrat 68, par rapport au sens de circulation du flux d'air, en vis-à-vis sont traversées par des orifices de 66 circulation d'air, ces orifices 66 pouvant être tronconiques et à section augmentant vers l'aval comme indiqué précédemment (figures 25 et 26). Ces deux faces 106, 108 parallèles l'une à l'autre sont reliées l'une à l'autre par des faces de jonction. Une première 110 et une seconde 112 faces de jonction sont parallèles l'une à l'autre et sont agencées en vis-à-vis l'une de l'autre. La première face de jonction 110 et la seconde face de jonction 112 sont reliées par une troisième face de jonction 114. Enfin, le substrat 68 comprend une quatrième face de jonction 116 sensiblement parallèle à la troisième face de jonction 114. Cette quatrième face de jonction 116 est reliée à la première face de jonction 110 par une face inclinée ou rampe 98 et est reliée à la deuxième face de jonction 112 par deux faces 102, 118 successives d'un renfoncement 100 dont l'une des faces 102 forme un épaulement.

L'utilisation du dispositif 72 selon l'invention s'effectue de la manière suivante. Un opérateur introduit un substrat 68 dans le chemin de déplacement en agençant la première face de jonction 110 du substrat 68 en vis-à-vis de la première ouverture 76. Le substrat 68 est poussé à l'intérieur du chemin de déplacement 74, la lame élastique anti-retour vient en appui sur la rampe 98, plus précisément la portion coudée ou sommet du V. Ladite portion coudée vient ensuite en appui sur la quatrième face de jonction 116 puis se relâche dans le renfoncement 100, l'extrémité libre 96 de la lame élastique anti-retour 88 venant se positionner en vis-à-vis de la surface de butée formée par l'épaulement 102, ce qui assure un blocage du substrat 68 dans la première position de stockage P₁. On comprend que l'introduction d'un substrat 68 dans la première ouverture 76 provoque de manière simultanée la sortie d'un substrat 68 de la seconde ouverture 78, cette sortie étant freinée par l'appui élastique de la portion coudée ou sommet de la lame de freinage 90 sur la rampe 98, ce qui permet de limiter la vitesse d'éjection du substrat 68 dans la seconde position de stockage P₂ de la seconde ouverture 78.

Il est à noter que le substrat 68 décrit en référence aux figures 25 et 26 est tout à fait adapté à être utilisé avec un porte-substrat 48 tel que décrit en référence à la figure 9.

Dans la présente description, le terme substrat 68 fait référence à une cartouche qui pourrait avoir tout type de forme de sorte que l'invention n'est pas limitée aux seules formes de cartouches décrites. En effet, le substrat pourrait avoir, selon un plan de coupe passant entre une face amont et une face aval et par la première extrémité et la seconde extrémité, une forme, circulaire, polygonal ou encore celle d'un parallélogramme, les faces amont et aval du substrat pouvant être planes et parallèles l'une à l'autre ou encore inclinées l'une par rapport à l'autre. Ces faces amont et aval pourraient encore ne pas être planes et avoir des formes courbes plus complexes.

De même, la rampe pourrait avoir en section une forme rectiligne ou encore concave ou convexe selon la résistance que l'on souhaite que l'opérateur exerce sur l'organe élastique anti-retour. La rampe peut avoir selon un plan perpendiculaire au plan de section précitée une forme rectiligne ou encore être incurvée concave ou convexe.

## Revendications

1. Dispositif de diffusion (10, 72) d'une substance volatile comprenant :
- un boîtier (12) comprenant intérieurement un circuit de circulation d'air entre au moins une entrée d'air (26) et au moins une sortie d'air (42a, 42b, 42c),
- un moyen de ventilation (44) agencé dans le circuit d'air et apte à faire circuler un flux d'air depuis l'entrée jusqu'à la sortie d'air, et
- une zone de réception (50, 70) agencée dans le circuit d'air en aval du moyen de ventilation et destinée à recevoir un substrat (46, 68) retenant une substance volatile,
**caractérisé en ce que** le circuit d'air comprend au moins trois chambres (54a, 54b, 54c) qui sont indépendantes fluidiquement les unes des autres, chacune des chambres étant agencée en aval de ladite zone de réception (50) et communiquant en aval avec sa propre sortie d'air (42a, 42b, 42c) différente des autres sorties d'air, une première (54a) et une deuxième (54b) chambres communiquant avec des sorties d'air (42a, 42b) projetant de l'air dans des sens opposés, et une troisième chambre (54b) communiquant avec une sortie d'air (42c) qui projette de l'air dans une direction perpendiculaire à la direction de projection d'air de sortie des première (54a) et deuxième (54b) chambres.

2. Dispositif selon la revendication 1, **caractérisé en ce que** la section des chambres (54a, 54b, 54c) augmente de l'amont vers l'aval.

3. Dispositif de diffusion selon la revendication 1 ou 2, **caractérisé en ce que** le boîtier (12) comprend une première paroi (14) de fond et une seconde paroi (16) entre lesquelles s'étendent au moins une première, une deuxième et une troisième parois latérales (18, 20, 22), un bord de chacune des première deuxième et troisième parois latérales (18, 20, 22) définissant avec un bord périphérique de l'une des première (14) et seconde (16) parois de fond, respectivement une fente de sortie d'air (42a, 42b, 42c) des première, deuxième et troisième chambres (54a, 54b, 54c).

4. Dispositif selon l'une des revendications 1 à 3, **caractérisé en ce qu'**il comprend :
- un chemin de déplacement (74) du substrat (68) formé à l'intérieur du boîtier (12) et s'étendant entre une première ouverture (76) et une seconde ouverture (78) formées dans des parois (18, 22) du boîtier (12) et accessibles depuis l'extérieur du boîtier (12), et
- des moyens de guidage (80) à déplacement du substrat (68) le long du chemin de déplacement (74) lequel intercepte le circuit d'air au niveau de la zone de réception (70).

5. Dispositif selon la revendication 4, **caractérisé en ce que** le chemin de déplacement (74) est dimensionné de manière à loger au moins trois substrats (68) agencés bout à bout dans respectivement une première position de stockage (P₁), une position de service (P_{S}) dans laquelle le substrat (68b) est dans la zone de réception, et une seconde position de stockage (P₂).

6. Dispositif selon la revendication 3 en combinaison avec la revendication 4 ou 5, **caractérisé en ce que** la première ouverture (76) est formée dans la première paroi latérale (18) et la seconde ouverture (78) est formée dans la deuxième paroi latérale (22).

7. Dispositif selon l'une des revendications 4 à 6, **caractérisé en ce que** les moyens de guidage (80) comprennent un rail rectiligne en U s'étendant entre la première ouverture (76) et la seconde ouverture (78) et délimitant intérieurement le chemin de déplacement (74) d'un substrat (68).

8. Dispositif selon l'une des revendications 4 à 7, **caractérisé en ce que** le chemin de déplacement (74) comprend au niveau de la première ouverture (76) un organe élastique anti-retour autorisant l'introduction d'un substrat (68) dans la première ouverture (76) et empêchant son retrait du chemin de déplacement (74) au travers de la première ouverture (76).

9. Dispositif selon l'une des revendications 4 à 8, **caractérisé en ce que** le chemin de déplacement comprend au niveau de la seconde ouverture (78) un organe élastique de freinage (90) configuré pour freiner la sortie d'un substrat (68) par la seconde ouverture (78).

10. Dispositif selon la revendication 8 et la revendication 9, **caractérisé en ce que** le rail est coiffé par un couvercle rectiligne, de préférence en U, portant à une extrémité l'organe élastique anti-retour et à une autre extrémité l'organe élastique de freinage.

11. Ensemble comprenant un dispositif selon l'une des revendications précédentes et un substrat (46, 68) conformé de manière à pouvoir être agencé dans ladite zone de réception (50, 70).

12. Ensemble selon la revendication 11, **caractérisé en ce que** le substrat (46, 68) a une forme parallélépipédique et comprend une pluralité de orifices (66) débouchant sur deux faces opposées (106, 108) du substrat (46, 68), au moins certains des orifices (66) ayant une section se réduisant depuis une extrémité vers l'autre extrémité des orifices, le substrat (46, 68) étant agencé dans la zone de réception (50, 70) de sorte que la section des orifices se réduit de l'amont vers l'aval.

13. Ensemble selon la revendication 11 ou 12, **caractérisé en ce que** le substrat (68) présente une section de forme générale rectangulaire comportant à un premier sommet (99) une rampe (98) inclinée en oblique et à un second sommet (104) un renfoncement (102).

14. Ensemble selon l'une des revendications 11 à 13, **caractérisé en ce que** le substrat (46, 68) est solide, intègre une substance volatile et comprend une face amont (106) et une face aval (108) en vis-à-vis entre lesquelles s'étendent des orifices (66) pour la circulation d'air au travers du substrat, le substrat (68) comportant une première extrémité (C) d'insertion dans un dispositif de diffusion et une seconde extrémité (D) opposée, la première extrémité (C) comprenant la rampe (98) destinée à coopérer avec un organe élastique anti-retour et la seconde extrémité (D) comprenant le renfoncement (100) dont une surface (102) est destinée à former une surface de butée de l'organe anti-retour.

15. Ensemble selon la revendication 14, **caractérisé en ce que** selon un plan de coupe intercalé entre les faces amont (106) et aval (108) et passant par les première (C) et seconde (D) extrémités, le substrat (68) a une forme de parallélogramme, la rampe (98) étant située au niveau d'un premier sommet (99) agencé du côté de la première extrémité (C) du substrat et le renfoncement (100) étant situé au niveau d'un second sommet (104) agencé du côté de la seconde extrémité (D) du substrat (68).

16. Ensemble selon la revendication 15, **caractérisé en ce que** le premier sommet (99) et le second sommet (104) sont reliés par une même arête s'étendant entre les première et seconde extrémités du substrat (68) et/ou **en ce que** la rampe (98) est plane et inclinée en oblique.

## Patentansprüche

1. Verteilungsvorrichtung (10, 72) für eine flüchtige Substanz, welche Folgendes umfasst:
- ein Gehäuse (12), welches im Innern einen Luftzirkulationskreis zwischen mindestens einem Lufteinlass (26) und mindestens einem Luftauslass (42a, 42b, 42c) umfasst,
- ein Belüftungsmittel (44), das im Luftkreis angeordnet ist und einen Luftstrom vom Lufteinlas bis zum Luftauslass zirkulieren lassen kann, und
- einen im Luftkreis vor dem Belüftungsmittel angeordneten Aufnahmebereich (50, 70), der dazu bestimmt ist, ein Substrat (46, 68) aufzunehmen, das eine flüchtige Substanz enthält,
- **dadurch gekennzeichnet, dass** der Luftkreis mindestens drei Kammern (54a, 54b, 54c) umfasst, die fluidisch unabhängig voneinander sind, wobei jede der Kammern stromabwärtig hinter dem besagtem Aufnahmebereich (50) angeordnet ist und stromabwärtig mit ihrem eigenen, von den anderen Luftauslässen abweichenden Luftauslass (42a, 42b, 42c) verbunden ist, eine erste (54a) und eine zweite (54b) Kammer, die mit den Luftauslässen (42a, 42b) verbunden sind, welche Luft in entgegengesetzter Richtung ausstoßen, und eine dritte Kammer (54b), die mit einem Luftauslass (42c) verbunden ist und Luft in einer Richtung senkrecht zur Luftausstoßrichtung der ersten (54a) und zweiten (54b) Kammer ausstößt.

2. Vorrichtung nach Anspruch 1, **dadurch gekennzeichnet, dass** der Querschnitt der Kammern (54a, 54b, 54c) in stromabwärtiger Richtung größer wird.

3. Verteilungsvorrichtung nach Anspruch 1 oder 2, **dadurch gekennzeichnet, dass** das Gehäuse (12) eine erste Rückwand (14) und eine zweite Wand (16) umfasst, zwischen denen sich zumindest eine erste, eine zweite und eine dritte Seitenwand (18, 20, 22) erstrecken, wobei ein Rand jeder der ersten, zweiten und dritten Seitenwände (18, 20, 22), welche mit einem umlaufenden Rand der ersten (14) oder der zweiten (16) Rückwand jeweils einen Luftaustrittsschlitz (42a, 42b, 42c) der ersten, zweiten und dritten Kammer (54a, 54b, 54) abgrenzen.

4. Vorrichtung nach einem der Ansprüche 1 bis 3, **dadurch gekennzeichnet, dass** sie Folgendes umfasst:
- eine Bewegungsbahn (74) des Substrats (68), die im Innern des Gehäuses (12) geformt ist und sich zwischen einer ersten Öffnung (76) und einer zweiten Öffnung (78) erstreckt, die in den Wänden (18, 22) des Gehäuses (12) geformt und vom Außenbereich des Gehäuses (12) aus zugänglich sind, und
- Führungsmittel (80) zum Bewegen des Substrats (68) entlang der Bewegungsbahn (74), welche den Luftkreis im Bereich des Empfangsbereichs (70) abfängt.

5. Vorrichtung nach Anspruch 4, **dadurch gekennzeichnet, dass** die Bewegungsbahn (74) so bemessen ist, dass zumindest drei Substrate (68) in jeweils einer ersten Lagerposition (P1), einer Betriebsposition (PS), in welcher sich das Substrat (68b) im Aufnahmebereich befindet, und einer zweiten Lagerposition (P2) aneinandergefügt angeordnet werden können.

6. Vorrichtung nach Anspruch 3 in Kombination mit Anspruch 4 oder 5, **dadurch gekennzeichnet, dass** die erste Öffnung (76) in der ersten Seitenwand (18) und die zweite Öffnung (78) in der zweiten Seitenwand (22 gebildet wird.

7. Vorrichtung nach einem der Ansprüche 4 bis 6, **dadurch gekennzeichnet, dass** die Führungsmittel (80) eine geradlinige, u-förmige Schiene umfassen, die sich zwischen der ersten Öffnung (76) und der zweiten Öffnung (78) erstreckt und innen die Bewegungsbahn (74) eines Substrats (68) begrenzt.

8. Vorrichtung nach einem der Ansprüche 4 bis 7, **dadurch gekennzeichnet, dass** die Bewegungsbahn (74) in Höhe der ersten Öffnung (76) ein elastisches Rückschlagelement umfasst, welches das Einführen eines Substrats (68) in die erste Öffnung (76) ermöglicht und dessen Entnahme von der Bewegungsbahn (74) durch die erste Öffnung (76) verhindert.

9. Vorrichtung nach einem der Ansprüche 4 bis 8, **dadurch gekennzeichnet, dass** die Bewegungsbahn in Höhe der zweiten Öffnung (78) ein elastisches Bremselement (90) umfasst, das so gestaltet ist, dass der Austritt eines Substrats (68) durch die zweite Öffnung (78) gebremst wird.

10. Vorrichtung nach Anspruch 8 und Anspruch 9, **dadurch gekennzeichnet, dass** die Schiene durch einen geradlinigen Deckel, vorzugsweise in u-Form, bedeckt ist, welcher an einem Ende das elastische Rückschlagelement und an einem anderen Ende das elastische Bremselement trägt.

11. Einheit, welche eine Vorrichtung nach einem der vorhergehenden Ansprüche umfasst und ein Substrat (46,68), das so ausgebildet ist, dass es in besagtem Aufnahmebereich (50, 70) angeordnet werden kann.

12. Einheit nach Anspruch 11, **dadurch gekennzeichnet, dass** das Substrat (46, 68) eine Quaderform hat und eine Vielzahl an Löchern (66) umfasst, die zu zwei gegenüberliegenden Seiten (106, 108) des Substrats (46,68) führen, wobei zumindest einige der Löcher (66) einen Querschnitt aufweisen, der sich von einem Ende zum anderen Ende der Löcher hin verschmälert, wobei das Substrat (46, 68) so im Aufnahmebereich (50,70) angeordnet ist, dass sich der Querschnitt der Löcher in stromabwärtiger Richtung verkleinert.

13. Einheit nach Anspruch 11 oder 12, in der eine Verteilungsvorrichtung nach Anspruch 4 benutzt wird, **dadurch gekennzeichnet, dass** das Substrat (68) einen generell rechteckigen Querschnitt aufweist, und an einem ersten Scheitelpunkt (99) eine schiefe Steigung (98) und an einem zweiten Scheitelpunkt (104) eine Vertiefung (102) umfasst.

14. Einheit nach einem der Ansprüche 11 bis 13, **dadurch gekennzeichnet, dass** das Substrat (46, 68) fest ist, eine flüchtige Substanz enthält und die stromaufwärtige Seite (106) und die gegenüberliegende stromabwärtige Seite (108) umfasst, zwischen denen sich die Öffnungen (66) zum Zirkulieren der Luft durch das Substrat erstrecken, und darüber hinaus ein erstes Ende (C) zum Einfügen in eine Verteilungsvorrichtung und ein zweites gegenüberliegendes Ende (D), wobei das erste Ende (C) die Steigung (98) umfasst, die dazu bestimmt ist, mit einem elastischen Rückschlagelement zusammen zu wirken, und das zweite Ende (D) die Vertiefung (100), von der eine Fläche (102) dazu bestimmt ist, eine Anschlagfläche für das Rückschlagelement zu bilden.

15. Einheit nach Anspruch 14, **dadurch gekennzeichnet, dass** auf einer Schnittebene zwischen der stromaufwärtigen Seite (106) und der stromabwärtigen Seite (108) das Substrat (68) beim Passieren des ersten (C) und des zweiten (D) Endes die Form eines Parallelogramms hat, wobei sich die Steigung (98) an einem ersten Scheitelpunkt (99) befindet, der an der Seite des ersten Endes (C) des Substrats angeordnet ist und sich die Vertiefung (100) an einem zweiten Scheitelpunkt (104) befindet, der an der Seite des zweiten Endes (D) des Substrats (68) angeordnet ist.

16. Einheit nach Anspruch 15, **dadurch gekennzeichnet, dass** der erste Scheitelpunkt (99) und der zweite Scheitelpunkt (104) durch ein und denselben Rand, der sich zwischen dem ersten und dem zweiten Ende des Substrats (68) erstreckt, miteinander verbunden sind, und/oder dadurch, dass die Steigung (98) eben und schräg abfallend ist.

## Claims

1. A device (10, 72) for diffusing a volatile substance, comprising:
- a housing (12) which internally comprises an air flow system between at least a air inlet (26) and at least a air outlet (42a, 42b, 42c),
- a ventilation means (44) arranged in the air circuit and capable of causing air to flow from the air inlet to the air outlet, and
- a receiving area (50, 70) arranged, in the air circuit, downstream of the ventilation means (44) and intended for receiving a substrate (46, 68) holding a volatile substance,
**characterized in that** the air circuit comprises at least three chambers (54a, 54b, 54c) which are fluidly independent of one another, with each one of the chambers being arranged downstream of said receiving area (50) and communicating, downstream, with its own air outlet (42a, 42b, 42c), different from the other air outlets, with a first one (54a) and a second one (54b) of the chambers communicating with air outlets (42a, 42b) which project air in opposite directions, and a third chamber (54c) communicating with a air outlet (42c) which projects air in a direction perpendicular to the air projection direction of the outlets of the first (54a) and second (54b) chambers.

2. A device according to claim 1, **characterized in that** the section of the chambers (54a, 54b, 54c) increases in the downstream direction.

3. A device according to claim 1 or 2, **characterized in that** the housing (12) comprises a first bottom wall (14) and a second wall (16) between which at least a first, a second and a third side walls (18, 20, 22) extend, with a edge of each one of the first, second and third side walls (18, 20, 22) respectively defining, with a peripheral edge of one of the first (14) and second (16) bottom walls, an air outlet slot (42a, 42b, 42c) of the first, second and third chambers (54a, 54b, 54c).

4. A device according to one of claims 1 to 3, **characterized in that** it comprises:
- a travel path (74) for the substrate (68) formed inside the housing (12) and extending between a first aperture (76) and a second aperture (78) formed in walls (18, 22) of the housing (12) and accessible from outside the housing (12), and
- means for guiding (80) the travel of the substrate (68) along the travel path (74) which intersects the air circuit at the receiving area (70).

5. A device according to claim 4, **characterized in that** the travel path (74) is so dimensioned as to accommodate at least three substrates (68) arranged end to end in respectively one first storage position (P₁), a service position (P_{S}) in which the substrate (68b) is in the receiving area, and a second storage position (P₂).

6. A device according to claim 3, when combined with claim 4 or 5, **characterized in that** the first aperture (76) is formed in the first side wall (18) and the second aperture (78) is formed in second side wall (22).

7. A device according to one of claims 4 to 6, **characterized in that** the guiding means (80) comprise a U-shaped rectilinear rail which extends between the first aperture (76) and the second aperture (78) and internally delimiting the travel path (74) of a substrate (68).

8. A device according to one of claims 4 to 7, **characterized in that** the travel path (74) comprises, at the first aperture (76), a non-return resilient member which enables the introduction of a substrate (68) through the first aperture (76) and preventing the removal thereof from the travel path (74) through the first aperture (76).

9. A device according to one of claims 4 to 8, **characterized in that** the travel path comprises, at the second aperture (78), a braking resilient member (90) so configured as to slow down the outcoming of one substrate (68) through the second aperture (78).

10. A device according to claim 8 and claim 9, **characterized in that** the rail is capped by a preferably U-shaped rectilinear cover, one end of which has the non-return resilient member and the other end of which has the braking resilient member.

11. An assembly comprising a device according to one of the preceding claims, and a substrate (46, 68) so formed as to be capable of being arranged in said receiving area (50, 70).

12. An assembly according to claim 11, **characterized in that** the substrate (46, 68) has a parallelepiped shape and comprises a plurality of holes (66) opening into two opposite faces (106, 108) of the substrate (46, 68), with at least some of the holes (66) having a section which decreases from one end to the other end of the holes, with the substrate (46, 68) being so arranged in the receiving area (50, 70) that the section of the holes decreases in the downstream direction.

13. An assembly according to claim 11 or 12, **characterized in that** the substrate (68) has a section with a generally rectangular shape including, at a first apex (99), an obliquely inclined ramp (98), and at a second apex (104), a recess (102).

14. An assembly according to one of claims 11 to 13, **characterized in that** the substrate (46, 68) is solid, incorporates a volatile substance and includes the upstream face (106) and the downstream face (108) facing each other between which the holes (66) are arranged for the circulation of air through the substrate, and further includes a first end for insertion (C) into a diffusion device and a second opposite end (D), with the first end (C) comprising the ramp (98) intended to cooperate with a non-return resilient member and the second end (D) comprising the recess (100), a surface (102) of which is intended to form an abutting surface for the non-resilient member.

15. An assembly according to claim 14, **characterized in that**, in a sectional plan coming in between the upstream (106) and downstream (108) faces and crossing the first (C) and second (D) ends, the substrate (68) has the shape of a parallelogram, with the ramp (98) being located at a first apex (99) arranged on the substrate first end (C) side and the recess (100) being located at a second apex (104) arranged on the substrate (68) second end (D) side.

16. An assembly according to claim 15, **characterized in that** the first apex (99) and the second apex (104) are connected by the same edge line which extends between the first and second ends of the substrate (68) and/or **in that** the ramp (98) is a flat surface and obliquely inclined.
